# EUROPEAN PATENT APPLICATION

(11) **EP 4 632 062 A1**
(43) Date of publication of application: **15.10.2025**
(21) Application number: 23892074.8
(22) Date of filing: 17.11.2023
(51) Int. Cl.: C12N 1/20, C12N 15/01, A61K 35/74, A61P 27/14, A23L 33/135, A61K 8/99, C12R 1/01

(54) **LACTIPLANTIBACILLUS PLANTARUM STRAIN, CULTURE MEDIUM DERIVED THEREFROM, AND ANTIALLERGIC USES THEREOF**

(30) Priority: 17.11.2022 KR 20220154863
(71) Applicant: Gi Biome Inc., Seongnam-si, Gyeonggi-do 13201 (KR)
(72) Inventor: JANG, Myung Ho, Seoul 05849 (KR); YANG, Bo Gie, Seoul 05849 (KR); KIM, A Ram, Namyangju-si, Gyeonggi-do 12095 (KR)
(74) Representative: Bryn Aarflot AS
(86) International application number: PCT/KR2023/018563
(87) International publication number: WO 2024/107012

(57) **Abstract**

The present disclosure relates to a novel microorganism, a lysate thereof, a culture broth thereof, and anti-allergy uses thereof, wherein a novel strain according to one aspect, a lysate derived from the strain, a culture broth of the strain, an extract of the culture broth, or a mixture thereof may be advantageously used in prevention, alleviation, or treatment of allergic diseases.

## Description

### Technical Field

The present disclosure relates to a novel microorganism, a lysate thereof, a culture broth thereof, an extract of the culture broth, and uses thereof for anti-allergic purposes.

### Background Art

A microbiome refers to the totality of microorganisms present in a given environment together with their entire genetic information, essentially representing a collection of genomes of individual organisms. Accordingly, the human microbiome indicates the microorganisms residing in and on the human body and all of their genetic information.

The human body coexists in a symbiotic relationship with numerous microbes, particularly in the intestines, which provide an optimal environment for nutrient intake and systematic colonization by the largest number of microorganisms. Intestinal microbes supply nutrients that cannot be produced by the host's enzymes alone, are deeply linked to the host's metabolism and immune system, and have been reported to be associated with various diseases such as irritable bowel syndrome, obesity, atopy, depression, rheumatoid arthritis, autism spectrum disorders, and dementia.

Allergy includes not only IgE-mediated allergic hypersensitivity but also non-IgE-mediated hypersensitivity, where the former involves IgE allergy antibodies causing allergic responses, and the latter arises from other immune components aside from IgE. Although the mechanism underlying non-IgE-mediated allergic diseases is not well understood, it is known that innate immunity and cell-mediated immunity (T-lymphocyte processes) are involved, with IgE antibodies themselves not substantially implicated. Activation of mast cells induces degranulation, leading to the secretion of histamine, cytokines, and chemokines stored within the granules, which in turn increase vascular permeability, contract smooth muscle, and enhance mucus secretion, thereby triggering a Type I allergic reaction.

These mast cells produce various cytokines through high-affinity IgE receptors (FcεRI), promoting the expression of inflammatory mediators and eliciting inflammatory allergic reactions. Among these, IL-4 is produced by T lymphocytes or mast cells, induces the production of IgE that triggers Type I allergic reactions, regulates cell-mediated immune responses, and has been reported to increase in actual patients (Ngoc et al., 2005). In addition, TNF-α is released from mast cells upon degranulation along with histamine and other inflammatory mediators when allergic reactions begin in the body, and induces the production of additional cytokines and inflammatory mediators, thereby exacerbating allergic and inflammatory responses (Chung and Barnes, 1999). Histamine, which already exists prior to the onset of an allergic reaction, is released externally once stimulated by an allergen (Schwartz and Huff, 1998).

Allergy is a condition arising from a malfunction of the immune system, wherein a substance that ordinarily has no impact on most people causes hypersensitivity reactions such as hives, itching, nasal discharge, and coughing, only in certain individuals. Allergy may be triggered by factors such as environmental pollution, Westernized diets and lifestyles, stress, heredity, and various allergens. The global incidence of allergies has been increasing, and 20 % to 25 % of the general population suffers from allergic disorders including rhinitis, asthma, atopic dermatitis, and food allergies. There is increasing evidence linking allergies to late-phase or chronic allergic reactions, suggesting that they play a role in the overall allergic inflammatory process.

Various methods have been proposed to treat allergic diseases, including allergen avoidance, administration of anti-allergic drugs, regulation of IgE synthesis in the body, and development of anti-IgE antibodies. However, these methods are often unable to address the fundamental causes of allergy, may have insufficient efficacy, or may induce serious side effects.

Hence, there is an urgent need for discovering anti-allergic active substances for developing anti-allergy therapeutics. To possess anti-allergic activity, it is insufficient merely to have therapeutic activity against IgE-mediated allergies; therapeutic activity against non-IgE-mediated allergies is also required.

Ongoing metagenome projects aimed at identifying gut microbial communities have revealed correlations between the distribution of intestinal flora and a variety of immune disorders, including allergies, and have clarified the close linkage among the diversity and distribution of intestinal microbes, gut immunity, and the immune system. Based on these findings regarding the gut microbial flora, it is necessary to develop a substance that has therapeutic activity not only against IgE-mediated allergies but also non-IgE-mediated allergies.

### Disclosure

### Technical Problem

One aspect is to provide a composition for inhibiting the production of Helper T Cells Type 2 (Th2)-mediated cytokines or alleviating allergic reactions, the composition including, as an active ingredient, a *Lactiplantibacillus plantarum* strain belonging to the genus *Lactiplantibacillus (Lactiplantibacillus* sp.).

Another aspect is to provide a *Lactiplantibacillus plantarum* strain GBCC_F0070, deposited under Accession No. KCTC15051BP, which belongs to the genus *Lactiplantibacillus (Lactiplantibacillus* sp.).

Another aspect is to provide a lysate derived from the strain, a culture broth derived from the strain, or an extract of the culture broth.

Another aspect is to provide a health functional food for preventing or alleviating an allergic disease, including, as an active ingredient, a *Lactiplantibacillus plantarum* strain, a lysate derived from the strain, a culture broth of the strain, or a mixture thereof.

Another aspect is to provide a pharmaceutical composition for treating or preventing an allergic disease, including, as an active ingredient, a *Lactiplantibacillus plantarum* strain, a lysate derived from the strain, a culture broth of the strain, or a mixture thereof.

Another aspect is to provide a cosmetic composition for preventing or alleviating an allergic skin condition, including, as an active ingredient, a *Lactiplantibacillus plantarum* strain, a lysate derived from the strain, a culture broth of the strain, or a mixture thereof.

Another aspect is to provide a method of preventing or treating an allergic disease, the method including administering a composition containing, as an active ingredient, an effective amount of a *Lactiplantibacillus plantarum* GBCC_F0070 strain (Accession No. KCTC15051BP), a culture of the strain, a lysate of the strain, or a mixture thereof, to a subject in need thereof.

Another aspect is to provide a use of a composition including a *Lactiplantibacillus plantarum* GBCC_F0070 strain (Accession No. KCTC15051BP), a culture of the strain, a lysate of the strain, or a mixture thereof, for preparing a pharmaceutical or health functional food preparation for preventing or treating an allergic disease.

### Technical Solution

One aspect provides a composition that inhibits the production of helper T cells type 2 (Th2)-mediated cytokines or alleviates allergic reactions, the composition including, as an active ingredient, a *Lactiplantibacillus plantarum* strain belonging to the genus *Lactiplantibacillus (Lactiplantibacillus* sp.).

Another aspect provides a *Lactiplantibacillus plantarum* strain, which belongs to the genus *Lactiplantibacillus (Lactiplantibacillus* sp.).

*Lactiplantibacillus* is a genus of aerobic or facultatively anaerobic, Gram-positive rod-shaped bacteria widely distributed in nature. Microorganisms belonging to the genus *Lactiplantibacillus* may include *Lactiplantibacillus plantarum, Lactiplantibacillus* fermentum, and the like. The present inventors conducted research to develop a novel strain with excellent anti-allergic effects and as a result, identified *Lactiplantibacillus plantarum* GBCC_F0070 as a candidate strain for anti-allergic activity. This strain was deposited with the Biological Resource Center of the Korea Research Institute of Bioscience and Biotechnology on August 16, 2022, under accession number KCTC15051 BP. The strain is a probiotic strain that is harmless to the human body, and may be used without side effects.

In an embodiment, the *Lactiplantibacillus plantarum* strain may be the strain deposited under Accession No. KCTC15051 BP.

In an embodiment, the *Lactiplantibacillus plantarum* strain may be a strain including 16S rRNA of SEQ ID NO: 1.

In an embodiment, the strain may be a strain having 16S rRNA including the nucleotide sequence of SEQ ID NO: 1 or a nucleotide sequence having at least 97% homology with the nucleotide sequence of SEQ ID NO: 1. Specifically, the strain may have a nucleotide sequence homology of at least 93 %, 95 %, 96 %, 97 %, 98 %, 99 %, 99.5 %, 99.8 %, 99.9 %, or 100 % with the nucleotide sequence of SEQ ID NO: 1 disclosed herein.

In an embodiment, the *Lactiplantibacillus plantarum* strain may comprise a recA gene sequence of SEQ ID NO: 2.

In an embodiment, the strain may have a recA gene including the gene sequence of SEQ ID NO: 2 or recA including at least 97 % gene sequence homology thereto. Specifically, the strain may have a nucleotide sequence homology of at least 93 %, 95 %, 96 %, 97 %, 98 %, 99 %, 99.5 %, 99.8 %, 99.9 %, or 100 % with the gene sequence of SEQ ID NO: 2 disclosed herein.

In an embodiment, the strain may be a naturally-occurring mutant.

In an embodiment, the strain may be a live bacterium, a heat-killed bacterium, an attenuated bacterium, or a cytoplasmic fraction obtained by lysing the strain, and is preferably a live bacterium.

In an embodiment, the strain may exhibit anti-allergic activity.

Specifically, without being bound by any particular theory, the allergic response mechanism may be divided into a first phase and a second phase.

In the first phase, an external allergen initially penetrates the body through epithelial cells, and the infiltrated allergen is then phagocytosed by antigen-presenting cells, which present fragments of it to T cells. When the T cells receive the presented antigen, they differentiate into Th2 cells (Helper T Cells Type 2) and produce Th2-mediated cytokines such as interleukin-4 (IL-4). These produced cytokines activate B cells, stimulating the production of immunoglobulin E (IgE) by B cells. The produced IgE antibodies bind to FcεRI receptors on the surface of mast cells, which is referred to as the atopic state, a prepared (primed) stage for allergic reactions.

In the second phase, the allergens previously that initially penetrated and were remembered by immune cells during the first phase reenter and crosslink with IgE bound on mast cells, and induce mast cell degranulation, thereby causing allergic inflammatory mediators such as histamine, leukotrienes, and tryptase present inside the mast cells to be released. These substances, with histamine being a representative example, may serve as direct triggers of various allergic inflammatory diseases. As described above, the mechanism of action of current therapeutic agents and materials for alleviating allergies often focus on the final step of the allergic reaction, namely inhibiting histamine release from mast cells or blocking histamine activity.

In an embodiment, the strain may have an activity of inhibiting the production of Th2-mediated cytokines, inhibiting mast cell degranulation, reducing serum IgE levels, or reducing serum MCPT-1 levels. Specifically, the inhibition of mast cell degranulation may involve inhibiting the secretion of β-hexosaminidase from activated mast cells.

In an embodiment, the strain may decrease the concentration of IgE in serum or reduce IgE binding to surface receptors on mast cells. Additionally, the strain may reduce MCPT-1 levels in serum.

Therefore, in an embodiment, the strain may be advantageously used to treat allergic diseases.

Another aspect provides a lysate derived from *Lactiplantibacillus plantarum,* a culture broth of the strain, an extract of the culture broth, or a mixture thereof.

In the present specification, the term "culture broth" is used interchangeably with "culture supernatant," "culture supernate," "conditioned culture broth," or "adjusted medium," and refers to the complete medium containing the strain, its metabolites, and residual nutrients, obtained by culturing the strain in a medium that provides nutrients for its growth and survival under in vitro conditions. The culture refers to a product obtained by culturing a probiotic strain in a known medium, and said product may or may not contain the strain itself. The medium may be selected from known liquid or solid media, for example MRS liquid medium, GAM liquid medium, MRS agar medium, GAM agar medium, and BL agar medium, but is not limited thereto.

The culture broth may include the culture broth itself obtained by culturing the strain, a concentrate thereof, or a freeze-dried product thereof, or may include a culture supernatant obtained by removing the strain from the culture broth, and/or a concentrate or freeze-dried product of the supernatant.

In the present specification, the term "lysate" is used interchangeably with "lysate product," and refers to an aqueous medium solution or suspension of disrupted microbial cells, such as *Lactiplantibacillus plantarum.* A cell lysate may include macromolecules such as DNA, RNA, proteins, peptides, carbohydrates, and lipids, and/or micromolecules such as amino acids, sugars, and fatty acids, or fractions thereof. Moreover, the lysate may include cellular debris, which can have a smooth or granular structure.

In the present specification, the term "culture broth extract" refers to an extract obtained from the culture broth or a concentrate thereof, and may include the extract itself, a dilution or concentrate of the extract, a dried product obtained by drying the extract, its adjusted or purified forms, or fractions thereof.

In an embodiment, the culture broth may be obtained by culturing *Lactiplantibacillus plantarum* in an appropriate medium (for example, MRS plate medium) at a temperature above 10 °C and below 40 °C for a certain duration, for example, 4 hours to 50 hours.

In an embodiment, the culture medium and culture conditions for culturing *Lactiplantibacillus plantarum* may be appropriately selected or modified by those skilled in the art.

Another aspect provides a composition including a lysate derived from a *Lactiplantibacillus plantarum* strain, a culture broth of the strain, an extract of the culture broth, or a mixture thereof.

In an embodiment, the composition may further include an additive selected from the group consisting of a preservative, a dye, an emulsifier, a sweetener, a stabilizer, a flavor enhancer, a flavoring agent, and an acidifier.

In an embodiment, the composition may be in one or more formulations selected from the group consisting of a solution, an emulsion, a viscous mixture, a powder, granules, tablets, and capsules.

In an embodiment, the composition may exhibit anti-allergic activity.

In an embodiment, the anti-allergic activity may include inhibiting the production of Th2-mediated cytokines, inhibiting mast cell degranulation, reducing serum IgE levels, reducing serum MCPT-1 levels, or inhibiting food allergy responses. Specifically, the inhibition of mast cell degranulation may involve inhibiting the secretion of β-hexosaminidase from activated mast cells.

In an embodiment, the Th2-mediated cytokine may be at least one selected from the group consisting of IL-4, IL-5, IL-6, IL-10, and IL-13.

In an embodiment, the composition may decrease the concentration of IgE in serum or reduce IgE binding to surface receptors on mast cells. In addition, the strain may reduce MCPT-1 levels in serum.

According to an embodiment, the anti-allergic activity may include inhibiting mast cell degranulation by 50 % to 70 % when the supernatant of *Lactiplantibacillus plantarum* GBCC_F0070 is administered to RBL-2H3 mast cells.

According to another embodiment, the anti-allergic activity may include inhibiting ear swelling by 30 % to 50 % in an MC903-induced atopic dermatitis animal model when the supernatant of *Lactiplantibacillus plantarum* GBCC_F0070 is administered.

According to another exemplary embodiment, the anti-allergic activity may include reducing the serum IgE concentration by 50 % to 70 % or reducing the serum MCPT-1 concentration by 50 % to 70 % when the supernatant of *Lactiplantibacillus plantarum* GBCC_F0070 is administered to an MC903-induced atopic dermatitis animal model.

Another aspect provides a use of *Lactiplantibacillus plantarum,* a lysate of the strain, a culture broth of the strain, or an extract of the culture broth for alleviating, preventing, or treating a disease.

In an embodiment, the use may include preventing, alleviating, or treating a disease or condition that is mediated or not mediated by Immunoglobulin E (IgE). Specifically, the disease mediated or not mediated by IgE may be an allergic disease.

In the present specification, "allergic disease" refers to a disease resulting from an excessive allergy response of the body's immune system to an external antigen, and may include one or more diseases selected from the group consisting of edema, anaphylaxis, allergic rhinitis, asthma, allergic conjunctivitis, allergic dermatitis, atopic dermatitis, contact dermatitis, urticaria, chronic spontaneous urticaria, hives, pruritus, anaphylactic shock, insect allergy, food allergy, drug allergy, and respiratory allergy, but is not limited thereto.

The onset of allergic disease may generally be divided into an early phase reaction and a late phase reaction. In the early phase reaction, after an antibody (IgE) is produced in response to antigen stimulation and binds to the high-affinity receptor (FcεRI) on the mast cell surface, reentry of the antigen leads to binding with the antibody (IgE) bound to FcεRI, rapidly transmitting signals within the mast cell and causing the secretion of inflammatory chemical mediators (cytokines, histamine, and leukotrienes) that induce allergies.

In the late phase reaction, Th2-type 2 cytokines (IL-4, IL-5, IL-6, IL-10, IL-13) induce the production of chemokines (monocyte chemoattractant protein-1, eotaxin-1, RANTES) by tissue fibroblasts or epithelial cells, resulting in the migration of inflammatory cells, particularly eosinophils, to the site of the allergic reaction. As a result, most allergic symptoms manifest as clinical signs such as dilation of capillaries around airway or skin epithelial cells, infiltration of immune cells, muscle contraction, and increased mucus secretion-leading to itching, edema, congestion, coughing, and phlegm-due to inflammatory mediators such as histamine secreted by mast cells and eosinophils.

In particular, among Th2-type 2 cytokines, IL-4 is produced by T cells and mast cells, playing roles in regulating B-cell class switching and promoting the production of IgE, whereas IL-5 influences the generation, activation, and survival of eosinophils, which are activated by IgE. Therefore, the activities of IL-4, IL-5, and eosinophils may be associated with an increase in IgE.

In an embodiment, the allergic disease may include allergic reactions caused by house dust, fungi, mites, or an animal's hair, dander, or excreta.

In an embodiment, the allergic disease may be a disease or condition that is either mediated or not mediated by immunoglobulin E (IgE) or may be a condition associated with an allergic disease.

In the present specification, the term "treat" or "treatment" may refer to the resolution of inflammation or bacterial infections in a shorter time compared to natural healing. The treatment may include improvement and/or alleviation of inflammation or bacterial infections. Additionally, the treatment may refer to the healing and/or recovering from symptoms triggered by inflammation or bacterial infections.

In the present specification, the phrase "includes as an active ingredient" means that the strain of the present disclosure, a lysate thereof, a culture broth thereof, or an extract of the culture broth is added in an amount sufficient to exhibit the aforementioned effects, and also encompasses that the strain of the present disclosure, a lysate thereof, a culture broth thereof, or an extract of the culture broth is formulated in various forms with various components added as minor ingredients for drug delivery and stabilization.

In an embodiment, the composition may include 0.001 wt% to 80 wt% of the *Lactiplantibacillus plantarum* strain, based on the total weight of the composition. In addition, the dose of the *Lactiplantibacillus plantarum* strain may be from 0.01 mg to 10,000 mg, 0.1 mg to 1,000 mg, 1 mg to 100 mg, 0.01 mg to 1,000 mg, 0.01 mg to 100 mg, 0.01 mg to 10 mg, or 0.01 mg to 1 mg. The strain may be incorporated into the composition at a therapeutically or nutritionally effective concentration. For example, the strain may be incorporated in an amount ranging from 10³ CFU/g to 10¹⁶ CFU/g, 10³ CFU/g to 10¹⁵ CFU/g, 10³ CFU/g to 10¹⁴ CFU/g, 10³ CFU/g to 10¹³ CFU/g, 10³ CFU/g to 10¹² CFU/g, 10⁴ CFU/g to 10¹⁶ CFU/g, 10⁴ CFU/g to 10¹⁵ CFU/g, 10⁴ CFU/g to 10¹⁴ CFU/g, 10⁴ CFU/g to 10¹³ CFU/g, 10⁴ CFU/g to 10¹² CFU/g, 10⁵ CFU/g to 10¹⁶ CFU/g, 10⁵ CFU/g to 10¹⁵ CFU/g, 10⁵ CFU/g to 10¹⁴ CFU/g, 10⁵ CFU/g to 10¹³ CFU/g, 10⁵ CFU/g to 10¹² CFU/g, 10⁶ CFU/g to 10¹³ CFU/g, 10⁶ CFU/g to 10¹² CFU/g, 10⁷ CFU/g to 10¹³ CFU/g, 10⁷ CFU/g to 10¹² CFU/g, 10⁸ CFU/g to 10¹³ CFU/g or 10⁸ to 10¹² CFU/g, or may be included in the composition as a culture containing an equivalent number of live or heat-killed bacteria. Specifically, for an adult patient, 1×10³ to 1×10¹⁶ CFU/g of live or heat-killed bacteria may be administered as a single dose or in divided doses. However, the dosage may vary depending on formulation method, route of administration, patient's age, weight, gender, pathological condition, diet, timing of administration, excretion rate, and responsiveness. A person skilled in the art may appropriately adjust the dosage considering the aforementioned factors. The administration frequency may be once a month, once every two weeks, once a week, once a day, twice a day, or three times a day, and specifically may be once, or twice or more within the range of clinically acceptable side effects. The administration may be to one site or to two or more sites. For animals other than humans, the same dosage as for humans per kg of body weight may be used, or for example, the above dosage may be adjusted based on the volume ratio (e.g., the average) of the target animal's organ (such as the heart) to that of a human. Possible routes of administration may include oral, sublingual, parenteral (for example, subcutaneous, intradermal, intravenous, intra-arterial, intramuscular, intraperitoneal, intradural, intra-articular, intrasynovial, intrasternal, or intralesional), rectal, topical (including transdermal), inhalation, and injection, or insertion of an implantable device or material. In an embodiment, the target animals may be a human or any other mammal, such as a monkey, mouse, rat, rabbit, sheep, cow, dog, horse, and pig. In an embodiment, the composition may include a heat-killed, dried strain that may be administered in an amount of 1 g to 10 g, 0.5 g to 1.5 g, 2.5 g to 3.5 g, or 4.5 g to 5.5 g, once to three times daily.

Another aspect provides a health functional food composition for preventing or alleviating an allergic disease, including, as an active ingredient, a *Lactiplantibacillus plantarum* strain, a lysate derived from the strain, a culture broth of the strain, or a mixture thereof.

The terms "strain", "anti-allergic activity", and "allergic disease" are as described above.

In an embodiment, the health functional food composition may exhibit anti-allergic activity.

In an embodiment, the anti-allergic activity may include inhibiting the production of a Th2-mediated cytokine, inhibiting mast cell degranulation, reducing serum IgE levels, or reducing serum MCPT-1 levels. Specifically, the inhibition of mast cell degranulation may involve inhibiting the secretion of β-hexosaminidase from activated mast cells.

In an embodiment, the Th2-mediated cytokine may be one or more selected from the group consisting of IL-4, IL-5, IL-6, IL-10, and IL-13.

In an embodiment, the health functional food composition may decrease the concentration of IgE in serum or reduce IgE binding to surface receptors on mast cells. Additionally, the strain may decrease MCPT-1 levels in serum.

The health functional food composition may be prepared in any one formulation selected from powders, granules, pills, tablets, capsules, candy, syrup, and beverages, but is not limited thereto. The health functional food composition of the present disclosure may be prepared by directly adding the active ingredient or by mixing the active ingredient with other food items or food ingredients, and may be appropriately prepared using conventional methods. Examples of food items to which the extract can be added may include any one form selected from caramels, meat, sausages, bread, chocolate, candy, snacks, cookies, pizza, ramen, other noodles, gum, ice cream, infant formula, milk, yogurt, cheese, fermented milk, milk powder, and other dairy products, soups, beverages, teas, drinks, alcoholic beverages, and vitamin complexes, and include all health functional foods in the usual sense. That is, there are no particular limitations on the types of food items. The health functional food composition may contain various nutrients, vitamins, minerals (electrolytes), synthetic and natural flavorings, colorants and enhancers (such as cheese and chocolate), pectic acid and salts thereof, alginic acid and salts thereof, organic acids, protective colloid thickeners, pH adjusters, stabilizers, preservatives, glycerin, alcohol, and carbonating agents used in carbonated drinks. Furthermore, the health functional food composition may contain fruit pulp for preparing natural fruit juice and vegetable beverages. The aforementioned components may be used either independently or in combination.

In an embodiment, the alleviation of allergic disease may include alleviation of immune hypersensitivity or alleviation of a skin condition caused by immune hypersensitivity.

In an embodiment, the alleviation of allergic disease may include inhibiting mast cell degranulation or reducing the levels of IgE or MCPT-1 in serum.

In an embodiment, the allergic disease may be selected from the group consisting of edema, anaphylaxis, allergic rhinitis, asthma, allergic conjunctivitis, allergic dermatitis, atopic dermatitis, contact dermatitis, urticaria, chronic spontaneous urticaria, hives, pruritus, anaphylactic shock, insect allergy, food allergy, drug allergy, and respiratory allergy.

In an embodiment, the allergic disease may include an allergic reaction caused by house dust, fungi, mites, or an animal's hair, dander, or excreta.

The health functional food composition may be used alone-containing only the strain or the culture broth thereof-or together with other food items or food ingredients, and may be appropriately used according to conventional methods. The amount of the active ingredient may be suitably determined according to the intended use (prevention, health maintenance, or therapeutic intervention). Generally, in the manufacture of foods or beverages, the composition disclosed herein may be added in an amount of up to 15 parts by weight relative to the raw material. There are no particular limitations on the types of the health functional food. Among types of health functional foods, beverage compositions may contain various flavoring agents or natural carbohydrates as additional ingredients, just like regular beverages. The natural carbohydrates may include monosaccharides such as glucose and fructose, disaccharides such as maltose and sucrose, polysaccharides such as dextrin and cyclodextrin, and sugar alcohols such as xylitol, sorbitol, and erythritol. As sweeteners, such as natural sweeteners (such as thaumatin and stevia extract) or synthetic sweeteners (such as saccharin and aspartame) may be used. The health functional food composition may additionally contain nutrients, vitamins, electrolytes, flavorings, colorants, pectic acid and salts thereof, alginic acid and salts thereof, organic acids, protective colloid thickeners, pH adjusters, stabilizers, preservatives, glycerin, alcohol, carbonating agents used in carbonated drinks, or a combination thereof. The health functional food composition may also contain pulp for making natural fruit juices, fruit juice beverages, and vegetable beverages, or a combination thereof.

Another aspect provides a pharmaceutical composition for treating or preventing allergic diseases, including, as an active ingredient, a *Lactiplantibacillus plantarum* strain, a lysate derived from the strain, a culture broth of the strain, or a mixture thereof.

The terms "strain," "anti-allergic activity," and "allergic disease" are as described above.

In an embodiment, the pharmaceutical composition may exhibit anti-allergic activity.

In an embodiment, the anti-allergic activity may include inhibiting the production of a Th2-mediated cytokine, inhibiting mast cell degranulation, reducing serum IgE levels, or reducing serum MCPT-1 levels. Specifically, the inhibition of mast cell degranulation may involve inhibiting the secretion of β-hexosaminidase from activated mast cells.

In an embodiment, the Th2-mediated cytokine may be one or more selected from the group consisting of IL-4, IL-5, IL-6, IL-10, and IL-13.

In an embodiment, the pharmaceutical composition may decrease the concentration of IgE in serum or reduce IgE binding to surface receptors on mast cells. In addition, the strain may decrease MCPT-1 levels in serum.

In an embodiment, the allergic disease may be selected from the group consisting of edema, anaphylaxis, allergic rhinitis, asthma, allergic conjunctivitis, allergic dermatitis, atopic dermatitis, contact dermatitis, urticaria, chronic spontaneous urticaria, hives, pruritus, anaphylactic shock, insect allergy, food allergy, drug allergy, and respiratory allergy.

In an embodiment, the allergic disease may include an allergic reaction caused by house dust, fungi, mites, or an animal's hair, dander, or excreta.

The pharmaceutical composition may further include a pharmaceutically acceptable diluent or carrier. The diluent may be lactose, corn starch, soybean oil, microcrystalline cellulose, or mannitol, and the lubricant may be magnesium stearate, talc, or a combination thereof. The carrier may be a filler, a disintegrant, a binder, a lubricant, or a combination thereof. The filler may be microcrystalline cellulose, lactose, low-substituted hydroxycellulose, or a combination thereof. The disintegrant may be carboxymethylcellulose calcium, sodium starch glycolate, anhydrous calcium hydrogen phosphate, or a combination thereof. The binder may be polyvinylpyrrolidone, low-substituted hydroxypropylcellulose, hydroxypropylcellulose, or a combination thereof. The lubricant may be magnesium stearate, silicon dioxide, talc, or a combination thereof.

The pharmaceutical composition may be formulated for oral or parenteral administration. Specifically, the pharmaceutical composition may be formulated for oral, rectal, intravenous, intravascular, intratumoral, subcutaneous, intradermal, or intraperitoneal delivery. More specifically, the oral formulation may be granules, powders, liquids, tablets, capsules, dry syrup, or a combination thereof, and the parenteral formulation may be an injectable preparation.

Another aspect provides a cosmetic composition for preventing or alleviating allergic disease, including, as an active ingredient, a *Lactiplantibacillus plantarum* strain, a lysate derived from the strain, a culture broth of the strain, or a mixture thereof.

The terms "strain," "anti-allergic activity," and "allergic disease" are as described above.

In an embodiment, the cosmetic composition may exhibit anti-allergic activity.

The cosmetic composition may have a formulation such as a skin-softening toner, a nourishing toner, a massage cream, a nourishing cream, an essence, a pack, a gel, an ampoule, or a skin-adhesive type cosmetic preparation.

Components included in the cosmetic composition may include not only the strain as an active ingredient but also components commonly used in cosmetic compositions, including conventional auxiliary agents and carriers such as stabilizers, solubilizers, vitamins, pigments, and fragrances.

Another aspect provides a method of preventing, alleviating, or treating a state of a subject, the method including administering or applying an effective amount of the aforementioned composition to a subject in need thereof.

In an embodiment, the state of the subject may be a disease or condition that is either mediated or not mediated by immunoglobulin E (IgE), or may be a state related to an allergic disease.

In the present specification, the term "administer" or "administration" refers to physically introducing a composition into a subject by any method or delivery system known to those skilled in the art. The administration may be carried out by methods known in the art. For example, the administration may be performed by any means that directly delivers the composition to the subject, such as intravenous, intramuscular, oral, transdermal, mucosal, intranasal, intratracheal, or subcutaneous routes. The administration may be performed systemically or locally.

The subject may be a mammal, such as a human, cow, horse, pig, dog, sheep, goat, or cat. The subject may be one requiring improvement in a condition related to inflammation or bacterial infection.

According to an embodiment, administration may involve administering from 0.00001 mg to 1,000 mg of the composition per individual per day, for example, 0.00001 mg to 500 mg, 0.00001 mg to 100 mg, 0.00001 mg to 50 mg, 0.00001 mg to 25 mg, 1 mg to 1,000 mg, 1 mg to 500 mg, 1 mg to 100 mg, 1 mg to 50 mg, 1 mg to 25 mg, 5 mg to 1,000 mg, 5 mg to 500 mg, 5 mg to 100 mg, 5 mg to 50 mg, 5 mg to 25 mg, 10 mg to 1,000 mg, 10 mg to 500 mg, 10 mg to 100 mg, 10 mg to 50 mg, or 10 mg to 25 mg. However, the dosage may vary depending on factors such as the formulation method, the route of administration, the patient's age, weight, gender, pathological condition, diet, time of administration, excretion rate, and responsiveness, and those skilled in the art may appropriately adjust the dosage in consideration of these factors. The administration frequency may be once a day or two or more times a day within a clinically acceptable range of side effects, and the composition may be administered to one site or multiple sites, daily or at 2- to 5-day intervals, for a total of 1 to 30 days per course of treatment. If necessary, the same treatment may be repeated after an appropriate period. For animals other than humans, the same dosage as for humans per kg of body weight may be used, or for example, the above dosage may be adjusted based on the volume ratio (e.g., the average) of the target animal's organ (such as the heart) to that of a human.

### Advantageous Effects

A novel strain according to one aspect, a lysate derived from the strain, a culture broth derived from the strain, an extract of the culture broth, or a mixture thereof may be advantageously used for the prevention, alleviation, or treatment of allergic diseases.

### Description of Drawings

FIG. 1 is a genome map of the GBCC_F0070 strain.
FIG. 2 is a graph illustrating the effect of GBCC_F0070 culture supernatant on inhibiting degranulation of RBL-2H3 mast cells.
FIG. 3 is a graph illustrating the effect of GBCC_F0070 on inhibiting ear swelling in an MC903-induced atopic dermatitis mouse model.
FIG. 4 is a graph illustrating the effect of GBCC_F0070 on reducing serum IgE levels in an MC903-induced atopic dermatitis mouse model.
FIG. 5 is a graph illustrating the effect of GBCC_F0070 on reducing serum MCPT-1 levels in an MC903-induced atopic dermatitis mouse model.
FIG. 6 is a graph illustrating the effect of *L. plantarum* GBCC_F0070 administration on inhibiting IgE-mediated allergic responses in a passive cutaneous anaphylaxis animal model.
FIG. 7 is a graph illustrating the effect of *L. plantarum* GBCC_F0070 administration on alleviating allergic responses in a food allergy animal model.
FIG. 8 is a graph illustrating the effect of *L. plantarum* GBCC_F0070 administration on reducing serum total IgE levels in a food allergy animal model.
FIG. 9 is a graph illustrating the effect of *L. plantarum* GBCC_F0070 administration on reducing inflammatory cells in bronchoalveolar lavage fluid (BALF) in an ovalbumin-induced asthma animal model.
FIG. 10 is a graph illustrating the effect of *L. plantarum* GBCC_F0070 administration on reducing serum total IgE levels in an ovalbumin-induced asthma animal model.
FIG. 11 is a graph illustrating the effect of *L. plantarum* GBCC_F0070 administration on inhibiting Th2 cytokines in serum in an ovalbumin-induced asthma animal model.
FIG. 12 is a graph illustrating the effect of *L. plantarum* GBCC_F0070 administration on inhibiting Th2 cytokines (IL-4, IL-5) in nasal mucosa in an ovalbumin-induced rhinitis animal model.
FIG. 13 is a graph illustrating the effect of *L. plantarum* GBCC_F0070 administration on reducing serum total IgE and ovalbumin-specific IgE levels in an ovalbumin-induced rhinitis animal model.

### Best Mode

Hereinafter, preferred embodiments are presented to aid in understanding the present disclosure. However, these embodiments are provided merely for illustrative purposes, and the subject matter of the present disclosure is not limited to these embodiments in any way. The embodiments may be modified in various manners and are not restricted to those explicitly disclosed below; rather, they may be implemented in numerous forms.

### Example 1. Isolation and Identification of Lactiplantibacillus plantarum Strain

### 1.1. Isolation of the Strain

The *Lactiplantibacillus plantarum* strain of the present disclosure was isolated from yeolmu kimchi (young radish kimchi) prepared in a home setting. After 23 days of fermentation, the yeolmu kimchi was serially diluted tenfold in PBS buffer and spread on MRS (deMan, Rogosa, Sharpe) agar plates, which are culture media for lactic acid bacteria. Following two days of incubation in an anaerobic chamber at 37 °C, bacterial colonies were observed. A single colony was then subcultured on fresh MRS agar plates to obtain a pure isolate. Identification of the isolated strain was performed using PCR and Sanger sequencing of the 16S rRNA gene. For long-term preservation of the strain, the culture in the late exponential phase was mixed with 30 % glycerol in a 1 : 1 ratio and stored in a deep freezer at -70 °C. After pure isolation and identification, further culturing was also conducted on MRS agar plates and liquid medium. When cultured on MRS solid medium for 48 hours, the strain formed large, round, white colonies with a smooth surface.

Through this process, the *Lactiplantibacillus plantarum* GBCC_F0070 strain (hereinafter also referred to as "GBCC_F0070") was ultimately selected.

### 1.2. Molecular Biological Identification of the Selected Strain

The identification of the *Lactiplantibacillus plantarum* GBCC_F0070 strain was performed based on sequence similarity of the 16S rRNA and recA genes. DNA was extracted from the bacterial culture using the FastDNA^{®} SPIN Kit for Soil (MP Bio, 6560200), and PCR was performed using the primer sequences listed in Table 1.

**[Table 1]**

| Gene | Primer | Sequence (5'-> 3') |
|---|---|---|
| 16S rRNA | 27F | AGAGTTTGATCMTGGCTCAG |
| | 1492R | TACGGYTACCTTGTTACGACTT |
| RecA | L. p_recA_F | CTGATGCACGGAAAGCAGC |
| | L. p_recA_R | CGCTTAAATGGYGGYGCMACC |

The sequence produced using the 27F and 1492R primers for amplifying the bacterial 16S rRNA gene is presented as Sequence ID No. 1. Based on the 16S rRNA sequence, GBCC_F0070 exhibited more than 98.5 % sequence similarity with 13 species within the genus *Lactiplantibacillus* ,including the type strain of *Lactiplantibacillus plantarum,* confirming that GBCC_F0070 belongs to one of these 13 species (Table 2).

**[Table 2]**

| Species | Standard strain name | Sequence similarity (%) |
|---|---|---|
| *Lactiplantibacillus pentosus* | DSM 20314(T) | 99.93 |
| *Lactiplantibacillus paraplantarum* | DSM 10667(T) | 99.86 |
| *Lactiplantibacillus argentoratensis* | DSM 16365(T) | 99.86 |
| *Lactiplantibacillus plantarum* | ATCC 14917(T) | 99.86 |
| *Lactiplantibacillus pingfangensis* | 382-1(T) | 99.23 |
| *Lactiplantibacillus nangangensis* | 381-7(T) | 99.15 |
| *Lactiplantibacillus herbarum* | TCF032-E4(T) | 99.09 |
| *Lactiplantibacillus fabifermentans* | DSM 21115(T) | 99.09 |
| *Lactiplantibacillus daowaiensis* | 203-3(T) | 99.09 |
| *Lactiplantibacillus daoliensis* | 116-1A(T) | 99.09 |
| *Lactiplantibacillus garii* | FI11369(T) | 99.02 |
| *Lactiplantibacillus xiangfangensis* | LMG 26013(T) | 98.95 |
| *Lactiplantibacillus plajomi* | NB53(T) | 98.67 |

Prior to the taxonomic revision of its genus name from *Lactobacillus plantarum* to *Lactiplantibacillus plantarum,* the strain had been designated as *Lactobacillus* plantarum. In addition, to achieve more accurate identification, beyond the 16S rRNA gene, sequence similarity of the recA gene, which is widely used as a bacterial taxonomic biomarker, was examined. As a result, it was confirmed that GBCC_F0070 is most closely related to the species *Lactiplantibacillus plantarum* (Table 3).

**[Table 3]**

| Species | Standard strain name | Sequence similarity (%) |
|---|---|---|
| *Lactiplantibacillus plantarum* | DSM 20174 | 99.86% |
| *Lactiplantibacillus argentoratensis* | DSM 16365 | 93.87% |
| *Lactiplantibacillus paraplantarum* | DSM 10667 | 87.32% |
| *Lactiplantibacillus pentosus* | DSM 20314 | 86.47% |
| *Lactiplantibacillus xiangfangensis* | LMG 26013 | 85.19% |
| *Lactiplantibacillus fabifermentans* | DSM 21115 | 83.90% |
| *Lactiplantibacillus herbarum* | TCF032-E4 | 83.62% |
| *Lactiplantibacillus daowaiensis* | 203-3 | 83.33% |
| *Lactiplantibacillus plajomi* | NBRC 107333 | 82.19% |
| *Lactiplantibacillus garii* | FI11369 | 81.77% |
| *Lactiplantibacillus nangangensis* | 381-7 | 81.34% |
| *Lactiplantibacillus daoliensis* | 116-1A | 81.05% |
| *Lactiplantibacillus pingfangensis* | 382-1 | 80.91% |

The recA gene sequence of GBCC_F0070 is presented as Sequence ID No. 2.

### Example 2. Genome and Comparative Genome Analysis of Lactiplantibacillus plantarum Strain

To identify the species and investigate the characteristics of the GBCC_F0070 strain based on its genome, the complete genome sequence of the strain was determined using next-generation sequencing (NGS) technology and bioinformatics tools, and the functions of the genes within the genome were inferred.

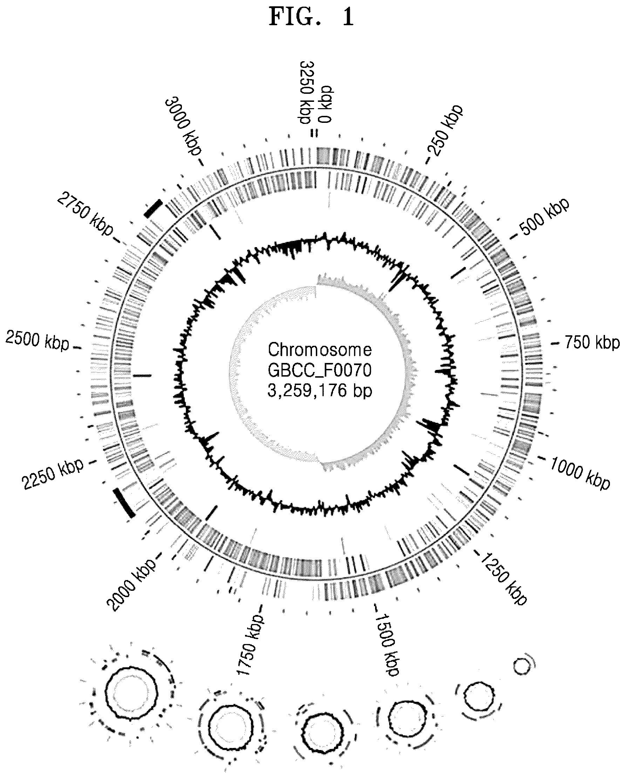

As a result of the genome sequencing, it was confirmed that GBCC_F0070 has one circular chromosome measuring 3,259,176 bp and six plasmids, and that GBCC_F0070 contains 3,140 CDSs, 16 rRNA genes, and 66 tRNA genes (FIG. 1, Table 4).

**[Table 4]**

| Characteristic | Value | % |
|---|---|---|
| No. of replicons | 1 | - |
| Genome size (bp) | 3,419,033 | 100.00 |
| DNA coding (bp) | 2,880,634 | 84.25 |
| DNA G+C (bp) | 1,513,087 | 44.25 |
| Total genes | 3,237 | 100.00 |
| Protein coding genes | 3,140 | 97.00 |
| RNA genes | 85 | 2.63 |
| rRNA genes | 16 | 0.49 |
| tRNA genes | 66 | 2.04 |
| Other ncRNA genes | 3 | 0.09 |
| Pseudo genes | 12 | 0.37 |
| Genes with function prediction | 2,652 | 81.93 |
| Genes assigned to COGs | 2,357 | 72.81 |
| Genes with Pfam domains | 2,483 | 76.71 |
| Genes with signal peptides | 176 | 5.44 |

Using the Jspecies program, the average nucleotide identity (ANI) was calculated between the genome of GBCC_F0070 and the genomes of related strains published in GenBank. The sequence similarity with the closest strain was 99.16 %, indicating that GBCC_F0070 is a novel strain not previously reported (Table 5).

**[Table 5]**

| Strain | Species | ANI value (%) |
|---|---|---|
| DSM 20174 | *Lactiplantibacillus plantarum* | 99.16 |
| DSM 16365 | *Lactiplantibacillus argentoratensis* | 94.99 |
| DSM 10667 | *Lactiplantibacillus paraplantarum* | 85.73 |
| DSM 20314 | *Lactiplantibacillus pentosus* | 79.41 |
| TCF032-E4 | *Lactiplantibacillus herbarum* | 76.98 |
| LMG 26013 | *Lactiplantibacillus xiangfangensis* | 76.06 |
| FI11369 | *Lactiplantibacillus garii* | 75.33 |
| DSM 21115 | *Lacticaseibacillus fabifermentans* | 75.31 |
| 116-1A | *Lactiplantibacillus daoliensis* | 74.60 |
| NBRC 107333 | *Lactiplantibacillus plajomi* | 74.77 |
| 381-7 | *Lactiplantibacillus nangangensis* | 74.44 |
| 203-3 | *Lactiplantibacillus daowaiensis* | 74.48 |
| 398-2 | *Lactiplantibacillus songbeiensis* | 74.38 |
| 218-3 | *Lactiplantibacillus dongliensis* | 74.16 |
| 382-1 | *Lactiplantibacillus pingfangensis* | 74.24 |
| NBRC 107235 | *Lactiplantibacillus modestisalitolerans* | 74.12 |

Accordingly, the inventors named the strain *"Lactiplantibacillus plantarum* GBCC_F0070" (accession number: KCTC 15051BP) and deposited it in the Korean Collection for Type Cultures (KCTC) at the Korea Research Institute of Bioscience and Biotechnology (KRIBB) on August 16, 2022.

### Experimental Example 1. Effect of Culture Supernatant of L. plantarum GBCC_F0070 on Inhibiting Degranulation of RBL-2H3 Mast Cells

To demonstrate therapeutic activity against IgE-mediated allergies, the inhibitory efficacy on mast cell degranulation was assessed. Culture supernatants from various *L. plantarum* strains, including *L. plantarum* GBCC_F0070, were each treated to activated mast cells that induce allergic reactions, and inhibitory effects on degranulation were screened through measurement of β-hexosaminidase secretion. β-hexosaminidase is stored in secretory granules of mast cells and releases histamine out of the cells in response to allergic reactions, thus may be used as an indicator of mast cell degranulation.

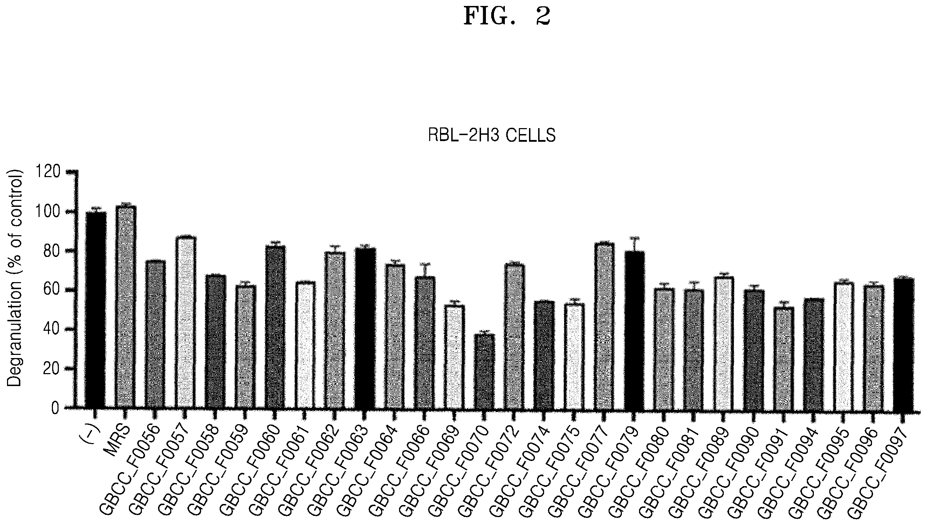

RBL-2H3 cells were suspended in 15 % FBS and MEM medium and dispensed into a 24-well plate at 1.5×10⁵ cells per well, and the cells were sensitized by culturing with 20 ng/mL DNP-IgE for about 12 hours. After the cells were washed once with PIPES buffer (25 mM PIPES, 119 mM NaCl, 5 mM KCl, 0.4 mM MgCl₂, 1 mM CaCl₂, 5.6 mM glucose, 0.1 % BSA), the culture supernatants from various *L. plantarum* strains were diluted in PIPES buffer in a ratio of 1 : 15 and pretreated to each well for 2 hours. The culture supernatants were obtained by culturing *L. plantarum* strains in MRS medium, separating the supernatant by precipitating the strains through centrifugation, and filtering the supernatant through a 0.22-µm filter. Subsequently, 25 ng/mL DNP-HSA(antigen) was added to stimulate the supernatant for 15 minutes, and the reaction was stopped by cooling on ice for 5 minutes. The supernatant was transferred to an e-tube and washed once with PIPES buffer, and the cells remaining on the plate were lysed with 0.1 % Triton X-100. 30 µL each of the supernatants and lysed cell samples were mixed with substrate buffer (1 mM 4-nitrophenyl N-acetyl-β-D-glucosaminide, 0.1 M sodium citrate) in a 96-well plate and allowed to react at 37 °C for 1 hour. The reaction was terminated by adding 0.1 M carbonate buffer, and absorbance at 405 nm was measured using a microplate reader, as shown in FIG. 2.

FIG. 2 is a graph illustrating the effect of GBCC_F0070 culture supernatant on inhibiting degranulation of RBL-2H3 mast cells.

As shown in FIG. 2, the culture supernatants of 26 different *L. plantarum* strains were screened, and the culture supernatant of *L. plantarum* GBCC_F0070 demonstrated about 61 % inhibition of mast cell degranulation, confirming its superior efficacy in inhibiting IgE-mediated allergic reactions.

### Experimental Example 2. Effect of L. plantarum GBCC_F0070 Strain Administration on Inhibiting Ear Swelling in MC903-induced Atopic Dermatitis Animal Model

The effect of *L. plantarum* GBCC_F0070 on inhibiting ear swelling was examined in an MC903-induced atopic dermatitis animal model.

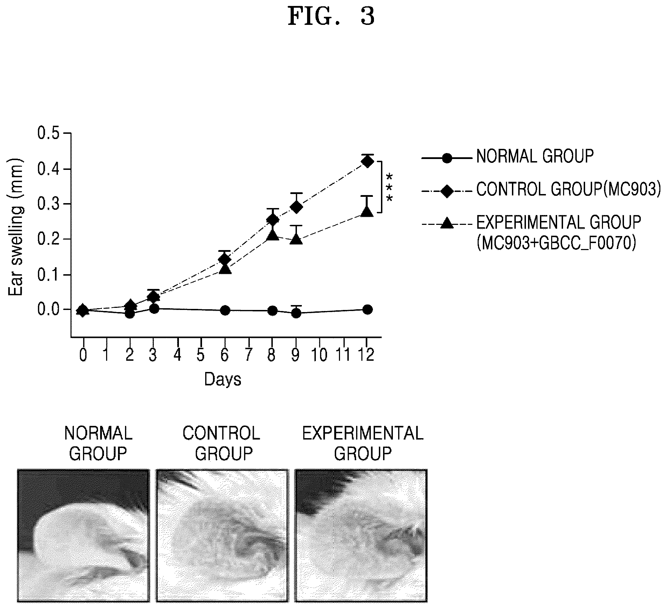

Six-week-old BALB/c mice were obtained and acclimated for one week. A freeze-dried *L. plantarum* GBCC_F0070 strain was suspended in D-PBS and orally administered at a dose of 2 × 10⁹ CFU per mouse daily, starting 7 days prior to atopic dermatitis induction and continuing until just before the end of the experiment. After restraining the mice to induce atopic dermatitis, the disease induction group was treated with 20 µL of 2 nmol MC903 (calcipotriol, C₂₇H₄₀O₃) dissolved in ethanol, applied to both ears daily for 12 days, while the normal group was treated with ethanol alone. Ear thickness was measured at the same time each day to assess atopic dermatitis-induced ear swelling, as shown in FIG. 3. The amount of change in ear swelling was calculated by subtracting the pre-induction ear thickness from the daily post-induction ear thickness.

FIG. 3 is a graph illustrating the effect of GBCC_F0070 on inhibiting ear swelling in a MC903-induced atopic dermatitis mouse model.

As shown in FIG. 3, in the atopic dermatitis model induced by MC903, the group (MC903+GBCC_F0070) administered with *L. plantarum* GBCC_F0070 exhibited about 34 % inhibition of ear swelling compared with the control group (MC903).

### Experimental Example 3. Effect of L. plantarum GBCC_F0070 Strain Administration on Reducing Serum Total IgE Levels in MC903-induced Atopic Dermatitis Animal Model

As overexpression of IgE is the most representative marker of atopic dermatitis, serum total IgE concentrations were measured using Mouse IgE ELISA Kit (BioLegend #432404) from the MC903-induced atopic dermatitis animal model in Experimental Example 3.

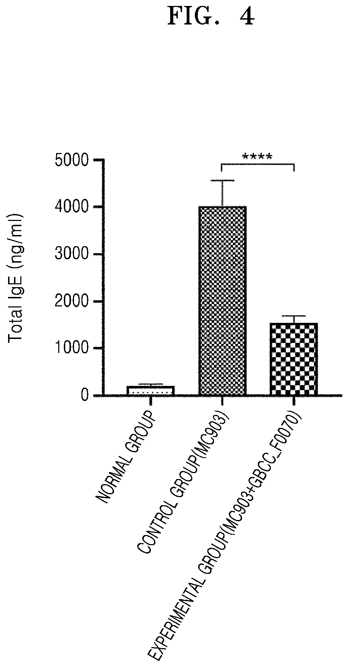

A capturing antibody diluted in coating buffer was allowed to react overnight at 4 °C in microwell plates. After four washes with wash buffer, the plates were blocked with assay diluent at room temperature for 1 hour. After four washes, standard samples and serum samples diluted 1 : 1,000 were added and allowed to react for two hours at room temperature. Then, after four washes, detection antibody was added and allowed to react for 1 hour at room temperature. After four washes, avidin-HRP was added and allowed to react for 30 minutes at room temperature, followed by five washes. TMB substrate solution was then added and allowed to react in the dark at room temperature for 20 minutes. Stop solution was added to each well to terminate the color reaction, and absorbance was measured at 450 nm using an ELISA reader. The results are shown in FIG. 4.

FIG. 4 is a graph showing the effect of GBCC_F0070 on reducing serum IgE levels in an MC903-induced atopic dermatitis mouse model.

As shown in FIG. 4, while the control group (MC903) showed a sharp increase in serum total IgE concentrations compared to the normal group, the experimental group (MC903+GBCC_F0070) administered with *L. plantarum* GBCC_F0070 showed a decrease of about 62 % in serum total IgE concentrations.

### Experimental Example 4. Effect of L. plantarum GBCC_F0070 Strain Administration on Reducing Serum MCPT-1 Levels in MC903-induced Atopic Dermatitis Animal Model

To assess the mast cell protease-1 (MCPT-1) concentration in serum from the MC903-induced atopic dermatitis animal model in Experimental Example 2, measurement was performed using Mouse MCPT-1 (mMCP-1) ELISA Kit (Invitrogen #88-7503).

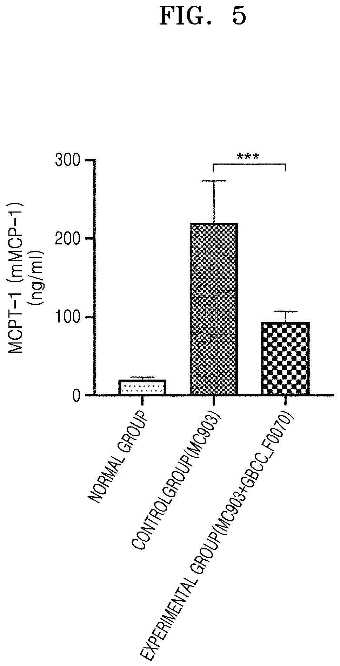

A capture antibody diluted in coating buffer was allowed to react overnight at 4 °C in microwell plates. After three washes with wash buffer, the plates were blocked with ELISA/ELISPOT diluent at room temperature for 1 hour. After three washes, standard samples and serum samples diluted 1 : 100 were added and allowed to react for two hours at room temperature. Then, after three washes, a detection antibody was added and allowed to react for 1 hour at room temperature. After three washes, avidin-HRP was added and allowed to react for 30 minutes at room temperature. After five washes, TMB substrate solution was added and allowed to react in the dark at room temperature for 15 minutes. Stop solution was added to each well to terminate the color reaction, and the absorbance was measured at 450 nm using an ELISA reader. The results are shown in FIG. 5.

FIG. 5 is a graph showing the effect of GBCC_F0070 on reducing serum MCPT-1 levels in an MC903-induced atopic dermatitis mouse model.

As shown in FIG. 5, while the control group (MC903) showed a sharp increase in serum MCPT-1 concentration compared to the normal group, the experimental group (MC903+GBCC_F0070) administered with *L. plantarum* GBCC_F0070 showed a decrease of about 57 % in serum MCPT-1 concentration.

### Experimental Example 5. Effect of L. plantarum GBCC_F0070 Strain Administration on Inhibiting IgE-Mediated Allergic Reactions in Passive Cutaneous Anaphylaxis Animal Model

The effect of *L. plantarum* GBCC_F0070 on inhibiting allergic reactions was evaluated using a passive cutaneous anaphylaxis (PCA) animal model induced by anti-DNP IgE.

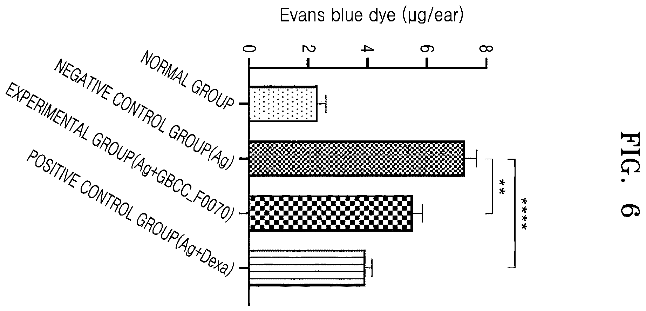

Four-week-old BALB/c mice were obtained and acclimatized for one week. A freeze-dried *L. plantarum* GBCC_F0070 strain was suspended in D-PBS and orally administered at a dose of 1 × 10¹⁰ CFU per mouse daily, starting two days prior to IgE sensitization and continuing until just before the end of the experiment. Dexamethasone (Dexa) was used as a positive control at a concentration of 10 mg/kg. To induce the passive cutaneous allergic reaction, 20 ng of anti-DNP IgE was injected locally into one ear of each mouse. After approximately 24 hours, 200 µL of DNP-HSA, diluted to 0.5 mg/mL in Evans blue dye solution (5 mg/mL), was injected into the tail vein. After 30 minutes, the mice were euthanized, and their ears were excised and placed in 700 µL of formamide. The samples were incubated at 63 °C for 12 hours to extract the dye exuded into the ear tissue, and the absorbance of the supernatant was measured at 620 nm. The amount of exuded Evans blue dye was quantified using a standard calibration curve, and the results are shown in FIG. 6.

FIG. 6 is a graph showing the effect of *L. plantarum* GBCC_F0070 on inhibiting IgE-mediated allergic responses in a PCA animal model.

As shown in FIG. 6, the experimental group treated with *L. plantarum* GBCC_F0070 (Ag+ GBCC_F0070) showed about 24 % inhibition of allergic responses compared to the negative control group (Ag) in the PCA model induced by anti-DNP IgE, confirming its anti-allergic effect.

### Experimental Example 6. Effect of L. plantarum GBCC_F0070 Strain Administration on Alleviating Allergic Symptoms in Food Allergy Animal Model

The effect of *L. plantarum* GBCC_F0070 on alleviating allergic symptoms was evaluated in a food allergy animal model induced by ovalbumin (OVA).

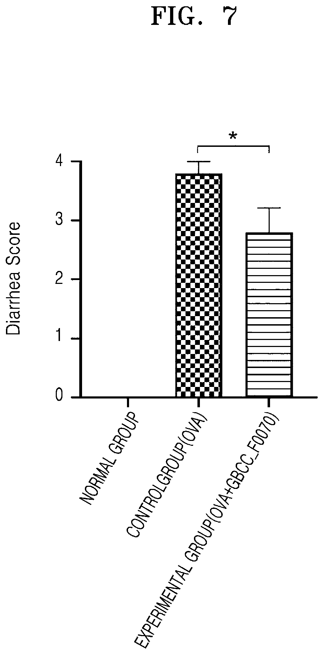

Five-week-old BALB/c mice were obtained and acclimated for one week. To induce food allergic responses, each mouse was sensitized by intraperitoneal injection of a mixture of 50 µg of OVA and 1 mg of alum hydroxide, administered a total of two times at two-week intervals. Two weeks after the final sensitization, allergic reactions were induced by oral administration of 50 mg of OVA five times at two-day intervals. To evaluate the therapeutic effect on allergies, a freeze-dried *L. plantarum* GBCC_F0070 strain was suspended in D-PBS and orally administered at a dose of 2 x 10⁹ CFU per mouse daily from the time of OVA sensitization until the end of the experiment. The fecal condition was visually observed to assess symptoms of food allergy, and diarrhea was scored based on severity. The results are shown in FIG. 7 and Table 6.

**[Table 6]**

| Diarrhea symptoms | Score |
|---|---|
| Normal | 0 |
| Soft stool | 1 |
| Soft stool/some diarrhea | 2 |
| Diarrhea | 3 |
| Severe watery diarrhea | 4 |

FIG. 7 illustrates the effect of *L. plantarum* GBCC_F0070 on alleviating allergic symptoms in a food allergy animal model. As shown in FIG. 7, the score for diarrhea symptoms was significantly increased in the control group (OVA) compared to the normal group, and in the food allergy model induced by OVA, the experimental group treated with *L. plantarum* GBCC_F0070 (OVA+GBCC_F0070) showed about 26 % alleviation in diarrhea symptoms compared to the control group.

### Experimental Example 7. Effect of L. plantarum GBCC_F0070 Strain Administration on Reducing Serum Total IgE Levels in Food Allergy Animal Model

Serum total IgE concentrations were measured in a food allergy animal model induced by ovalbumin using a Mouse IgE ELISA Kit (Biolegend #432404).

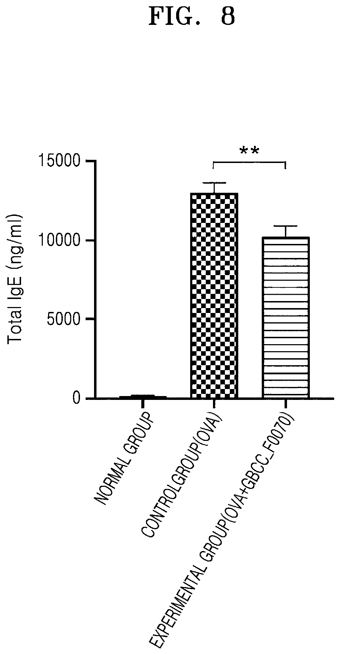

A capture antibody diluted in coating buffer and was incubated overnight at 4 °C in microwell plates. After four washes with wash buffer, the plates were blocked with assay diluent at room temperature for 1 hour. After four washes, standard samples and serum samples diluted 1 : 1,000 were added and allowed to react at room temperature for 2 hours. After another four washes, a detection antibody was added and allowed to react at room temperature for 1 hour. After four washes, avidin-HRP was added and allowed to react at room temperature for 30 minutes, followed by five washes. TMB substrate solution was then added and allowed to react for 20 minutes in the dark at room temperature. A stop solution was added to each well to terminate the color development reaction, and absorbance at 450 nm was measured using an ELISA reader. The results are shown in FIG. 8.

FIG. 8 is a graph showing the effect of *L. plantarum* GBCC_F0070 administration on reducing serum total IgE levels in a food allergy animal model.

As shown in FIG. 8, while the control group (OVA) showed a sharp increase in serum total IgE concentrations compared to the normal group, the experimental group treated with *L. plantarum* GBCC_F0070 (OVA+GBCC_F0070) showed a decrease of about 21 % in serum total IgE concentrations.

### Experimental Example 8. Effect of L. plantarum GBCC_F0070 Strain Administration on Reducing Inflammatory Cells in Bronchoalveolar Lavage Fluid (BALF) in Ovalbumin-Induced Asthma Animal Model

The asthma-alleviating effect of the *L. plantarum* GBCC_F0070 strain was assessed in an animal model of bronchial asthma induced by ovalbumin (OVA).

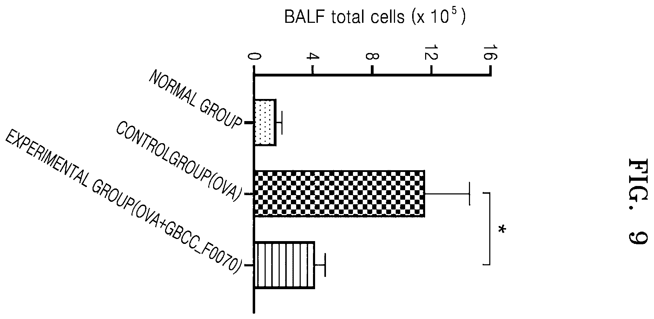

Five-week-old BALB/c mice were obtained and acclimated for one week. To establish an asthma mouse model, each mouse was sensitized by intraperitoneal injection of a mixture of 50 µg of OVA and 1 mg of alum hydroxide, administered a total of two times at two-week intervals. One week after the final sensitization, from Day 21 to Day 25, each mouse was anesthetized, and 150 µg of ovalbumin was instilled intranasally to induce pulmonary aspiration, thereby inducing asthma. Twenty-four hours after the final challenge, each mouse was anesthetized, and the trachea was excised. Bronchoalveolar lavage fluid was collected, and the total cell count therein was measured. The results are shown in FIG. 9.

FIG. 9 is a graph illustrating the effect of *L. plantarum* GBCC_F0070 strain administration on reducing inflammatory cells in bronchoalveolar lavage fluid (BALF) in an ovalbumin-induced asthma animal model.

As shown in FIG. 9, a marked increase in inflammatory cells in the bronchoalveolar lavage fluid was observed in the asthma-induced group compared with the normal group, and compared with the control group (OVA), the experimental group (OVA+GBCC_F0070) administered with *L. plantarum* GBCC_F0070 showed a decrease of about 64 % in inflammatory cells in the bronchoalveolar lavage fluid.

### Experimental Example 9. Effect of L. plantarum GBCC_F0070 Strain Administration on Reducing Serum Total IgE and Ovalbumin-Specific IgE Levels in Ovalbumin-Induced Asthma Animal Model

In order to assess the serum total IgE and ovalbumin (OVA)-specific IgE concentrations in an ovalbumin-induced asthma animal model, measurement was performed using a Mouse IgE ELISA Kit (Biolegend #432404) and a Mouse OVA-Specific IgE ELISA Kit (Biolegend #439807).

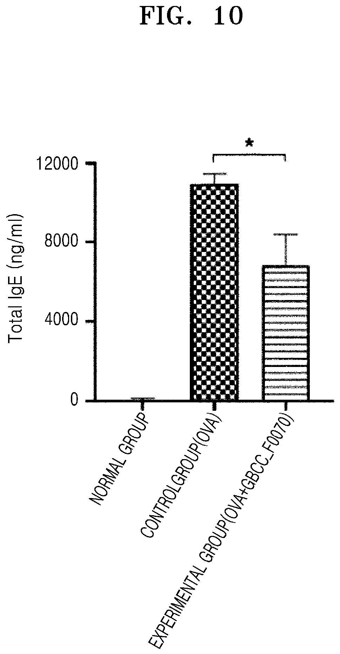

For serum total IgE, a capturing antibody diluted in coating buffer was incubated overnight at 4 °C in microwell plates. After four washes with wash buffer, the plates were blocked with assay diluent at room temperature for 1 hour. After four more washes, standard samples and serum samples diluted 1 : 500 (for total IgE) and 1 : 2 (for OVA-specific IgE) were added and allowed to react at room temperature for 2 hours. Subsequently, the plates were washed four times, a detection antibody was added, and allowed to react at room temperature for 1 hour. After four washes, avidin-HRP was added and allowed to react at room temperature for 30 minutes, followed by five washes. TMB substrate solution was then added and allowed to react for 20 minutes in the dark at room temperature. A stop solution was added to each well to terminate the color development reaction, and absorbance at 450 nm was measured using an ELISA reader. The results are shown in FIG. 10.

FIG. 10 is a graph illustrating the effect of *L. plantarum* GBCC_F0070 strain administration on reducing serum total IgE levels in an ovalbumin-induced asthma animal model.

As shown in FIG. 10, although the control group (OVA) showed a sharp increase in serum total IgE concentrations compared to the normal group, the experimental group (OVA+GBCC_F0070) administered with *L. plantarum* GBCC_F0070 showed a decrease of about 37 % in serum total IgE concentrations.

### Experimental Example 10. Effect of L. plantarum GBCC_F0070 Strain Administration on Inhibiting Th2 Cytokines in Serum in Ovalbumin-Induced Asthma Animal Model

To assess the serum IL-5 concentration in an ovalbumin-induced asthma animal model, measurement was performed using Mouse IL-5 ELISA Kit (Biolegend #431204).

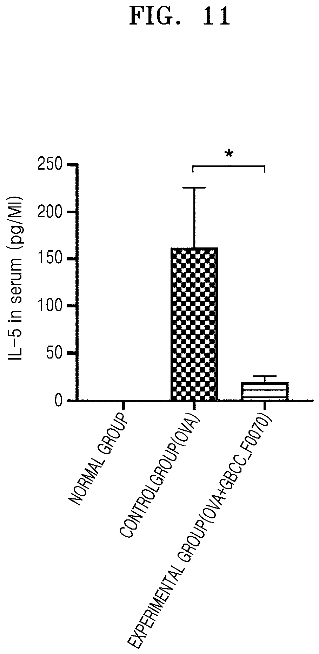

A capture antibody diluted in coating buffer was incubated overnight at 4 °C in microwell plates. After four washes with wash buffer, the plates were blocked with assay diluent at room temperature for 1 hour. After four washes, standard samples and serum samples diluted 1 : 5 were added and allowed to react at room temperature for 2 hours. After another four washes, a detection antibody was added and allowed to react at room temperature for 1 hour. After four washes, avidin-HRP was added and allowed to react at room temperature for 30 minutes, followed by five washes. TMB substrate solution was then added and allowed to react for 20 minutes in the dark at room temperature. A stop solution was added to each well to terminate the color development reaction, and the absorbance at 450 nm was measured using an ELISA reader. The results are shown in FIG. 11.

FIG. 11 is a graph showing the effect of *L. plantarum* GBCC_F0070 administration on inhibiting serum Th2 cytokines in an ovalbumin-induced asthma animal model.

As shown in FIG. 11, although the control group (OVA) showed a sharp increase in serum IL-5 concentration compared to the normal group, the experimental group (OVA+GBCC_F0070) administered with the *L. plantarum* GBCC_F0070 strain showed a statistically significant decrease of about 88 % in serum IL-5 production compared to the control group.

This result confirms that the *L. plantarum* GBCC_F0070 strain exerts an inhibitory effect on serum Th2 cytokines, indicating that the strain has therapeutic activity not only against IgE-mediated allergies but also against non-IgE-mediated allergies.

### Experimental Example 11. Effect of L. plantarum GBCC_F0070 Strain Administration on Inhibiting Th2 Cytokines (IL-4, IL-5, IL-13) in Nasal Mucosa in Ovalbumin-Induced Rhinitis Animal Model

The rhinitis-alleviating effect of the *L. plantarum* GBCC_F0070 strain was assessed in an animal model of rhinitis induced by ovalbumin (OVA).

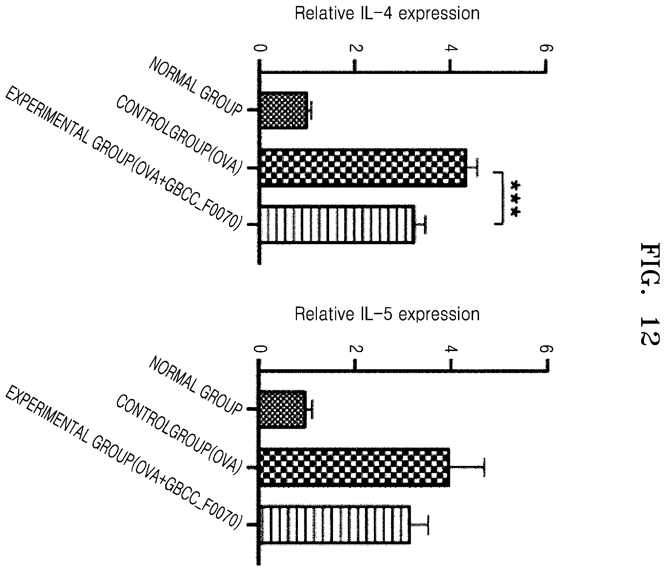

Five-week-old BALB/c mice were obtained and acclimatized for one week. To establish a rhinitis mouse model, each mouse was sensitized by intraperitoneal injection of a mixture of 50 µg of OVA and 2 mg of alum hydroxide, administered a total of three times at one-week intervals. One week after the final sensitization, from Day 21 to Day 26, rhinitis was induced by intranasal administration of 100 µg of ovalbumin without anesthesia to prevent aspiration of the antigen into the lungs. 24 hours after the final challenge, the nasal mucosa was excised and homogenized for RNA isolation. The isolated RNA was then synthesized into cDNA, and the mRNA expression levels of Th2 cytokines (IL-4, IL-5, and IL-13) were analyzed using a QuantStudio 3 Real-Time PCR Instrument. The results are shown in FIG. 12.

FIG. 12 is a graph showing the effect of *L. plantarum* GBCC_F0070 administration on inhibiting Th2 cytokines (IL-4 and IL-5) in the nasal mucosa in an ovalbumin-induced rhinitis animal model.

As shown in FIG. 12, a marked increase in Th2 cytokines in the nasal mucosa was observed in the rhinitis-induced group compared to the normal group. Compared to the control group (OVA), the experimental group (OVA+GBCC_F0070) administered with the *L. plantarum* GBCC_F0070 strain showed a statistically significant inhibition of about 25 % in IL-4 mRNA expression and a decrease of 21 % in IL-5 mRNA expression.

This result confirms that the *L. plantarum* GBCC_F0070 strain exerts an inhibitory effect on serum Th2 cytokines, indicating that the strain has therapeutic activity not only against IgE-mediated allergies but also against non-IgE-mediated allergies.

### Experimental Example 12. Effect of L. plantarum GBCC_F0070 Strain Administration on Reducing Serum Total IgE and Ovalbumin-Specific IgE Levels in Ovalbumin-Induced Rhinitis Animal Model

In order to assess serum total IgE and ovalbumin (OVA)-specific IgE concentrations in an ovalbumin-induced rhinitis animal model, measurement was performed using a Mouse IgE ELISA Kit (Biolegend #432404) and a Mouse OVA-Specific IgE ELISA Kit (Biolegend #439807).

For serum total IgE, a capturing antibody diluted in coating buffer was incubated overnight at 4 °C in microwell plates. After four washes with wash buffer, the plates were further blocked with assay diluent for 1 hour at room temperature. After four more washes, standard samples and serum samples diluted 1 : 500 (for total IgE) and 1 : 2 (for OVA-specific IgE) were added and allowed to react at room temperature for 2 hours. After four washes, a detection antibody was added and allowed to react at room temperature for 1 hour.

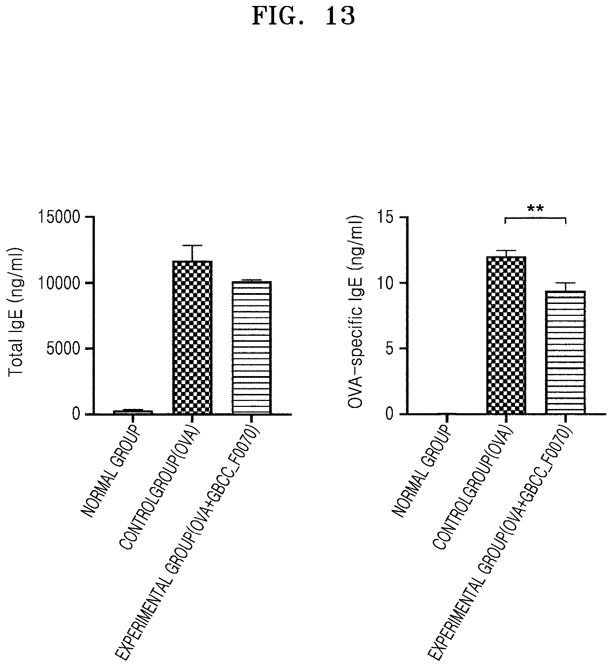

After four washes, avidin-HRP was added and allowed to react at room temperature for 30 minutes, followed by five washes. TMB substrate solution was then added and allowed to react in the dark at room temperature for 20 minutes. Stop solution was added to each well to terminate the color development reaction, and absorbance at 450 nm was measured using an ELISA reader. The results are shown in FIG. 13.

FIG. 13 is a graph showing the effect of *L. plantarum* GBCC_F0070 administration on reducing serum total IgE and ovalbumin-specific IgE levels in an ovalbumin-induced rhinitis animal model.

As shown in FIG. 13, although the control group (OVA) showed a sharp increase in serum total IgE concentrations compared to the normal group, the experimental group (OVA+GBCC_F0070) administered with the *L. plantarum* GBCC_F0070 strain showed a decrease of about 14 % in serum total IgE concentrations. Furthermore, compared to the control group, the experimental group administered with *L. plantarum* GBCC_F0070 showed a decrease of about 22 % in ovalbumin-specific IgE concentrations in serum.

These results demonstrate that the *L. plantarum* GBCC_F0070 strain can be advantageously used for preventing, alleviating, or treating atopic dermatitis or allergic diseases.

The above description of the present disclosure is solely for illustration, and it will be apparent to those skilled in the art that various alterations can be made without departing from the technical concept or essential features of the present disclosure. Accordingly, the embodiments and experimental examples described above should be understood to be illustrative in all respects, and not restrictive in any way.

### [Accession No]

Name of Depository Institution: Korea Research Institute of Bioscience and Biotechnology
Accession No: KCTC15051BP
Accession Date: August 16, 2022

## Claims

1. A composition for inhibiting the production of a Th2 (Helper T cells type 2)-mediated cytokine or alleviating an allergic reaction, the composition comprising, as an active ingredient, a *Lactiplantibacillus plantarum* strain belonging to the genus *Lactiplantibacillus (Lactiplantibacillus* sp.).

2. The *Lactiplantibacillus plantarum* GBCC_F0070 strain, belonging to the genus *Lactiplantibacillus (Lactiplantibacillus* sp.), deposited under Accession No. KCTC15051BP.

3. The strain of claim 2, wherein the strain comprises the 16S rRNA of SEQ ID NO: 1.

4. The strain of claim 2, wherein the strain comprises the recA gene sequence of SEQ ID NO: 2.

5. The strain of claim 2, wherein the strain comprises a naturally-occurring mutation of a *Lactiplantibacillus plantarum* strain.

6. The strain of claim 2, wherein the strain is a live bacterium, a heat-killed bacterium, an attenuated bacterium, or a cytoplasmic fraction obtained by lysing the strain.

7. The strain of claim 2, wherein the strain comprises the following properties:
- inhibiting the production of a Th2-mediated cytokine;
- inhibiting mast cell degranulation;
- reducing serum IgE levels; or
- reducing serum MCPT-1 levels.

8. The strain of claim 7, wherein the Th2-mediated cytokine is at least one selected from the group consisting of IL-4, IL-5, IL-6, IL-10, and IL-13.

9. A composition, comprising a lysate derived from the strain of claim 2, a culture broth of the strain, or an extract of the culture broth.

10. The composition of claim 9, wherein the composition is for preventing or treating a disease or condition mediated by IgE.

11. The composition of claim 9, wherein the composition decreases serum IgE levels or reduces IgE binding to surface receptors on mast cells.

12. The composition of claim 9, further comprising an additive selected from the group consisting of a preservative, a dye, an emulsifier, a sweetener, a stabilizer, a flavor enhancer, a flavoring agent, and an acidifier.

13. The composition of claim 9, wherein the composition is formulated in at least one form selected from the group consisting of a solution, an emulsion, a viscous mixture, a powder, granules, a tablet, and a capsule.

14. A health functional food composition for alleviating or preventing allergic disease, comprising, as an active ingredient, the *Lactiplantibacillus plantarum* GBCC_F0070 strain deposited under Accession No. KCTC15051BP, a lysate derived from the strain, a culture broth of the strain, or a mixture thereof.

15. The health functional food composition of claim 14, wherein the alleviation of allergic disease comprises:
- inhibiting the production of a Th2-mediated cytokine;
- inhibiting mast cell degranulation;
- reducing serum IgE levels; or
- reducing serum MCPT-1 levels.

16. The strain of claim 15, wherein the Th2-mediated cytokine is at least one selected from the group consisting of IL-4, IL-5, IL-6, IL-10, and IL-13.

17. The health functional food composition of claim 14, wherein the alleviation of allergic disease comprises alleviating immune hypersensitivity or improving a skin condition caused by immune hypersensitivity.

18. The health functional food composition of claim 14, wherein the allergic disease is selected from the group consisting of edema, anaphylaxis, allergic rhinitis, asthma, allergic conjunctivitis, allergic dermatitis, atopic dermatitis, contact dermatitis, urticaria, chronic spontaneous urticaria, hives, pruritus, anaphylactic shock, insect allergy, food allergy, drug allergy, and respiratory allergy.

19. The health functional food composition of claim 14, wherein the allergic disease comprises an allergic reaction caused by house dust, fungi, mites, or an animal's hair, dander, or excreta.

20. A pharmaceutical composition for treating or preventing an allergic disease, comprising, as an active ingredient, the *Lactiplantibacillus plantarum* GBCC_F0070 strain deposited under Accession No. KCTC15051BP, a lysate derived from the strain, a culture broth of the strain, or a mixture thereof.

21. The pharmaceutical composition of claim 20, wherein the composition has an activity of inhibiting mast cell degranulation.

22. The pharmaceutical composition of claim 20, wherein the composition (i) inhibits the expression of IgE in serum or (ii) inhibits the level of MCPT-1 in serum.

23. The pharmaceutical composition of claim 20, wherein the allergic disease is selected from the group consisting of edema, anaphylaxis, allergic rhinitis, asthma, allergic conjunctivitis, allergic dermatitis, atopic dermatitis, contact dermatitis, urticaria, chronic spontaneous urticaria, hives, pruritus, insect allergy, food allergy, drug allergy, and respiratory allergy.

24. The pharmaceutical composition of claim 20, wherein the allergic disease comprises an allergic reaction caused by house dust, fungi, mites, or an animal's hair, dander, or excreta.

25. The pharmaceutical composition of claim 20, wherein the composition is suitable for oral or parenteral administration.

26. The pharmaceutical composition of claim 25, wherein the oral or parenteral administration is performed once a month, once every two weeks, once a week, once a day, twice a day, or three times a day.

27. The pharmaceutical composition of claim 26, wherein the parenteral administration is a subcutaneous, intradermal, intravenous, arterial, intramuscular, intraperitoneal, intradural, intra-articular, intrasynovial, intrasternal, intralesional, or rectal administration.

28. The pharmaceutical composition of claim 20, wherein the composition is formulated for oral, rectal, intravenous, intravascular, intratumoral, subcutaneous, intradermal, or intraperitoneal delivery.

29. A cosmetic composition for alleviating or preventing an allergic skin condition, comprising the *Lactiplantibacillus plantarum* GBCC_F0070 strain deposited under Accession No. KCTC15051BP, a lysate derived from the strain, a culture broth of the strain, or a mixture thereof.

30. A method of preventing or treating an allergic disease, the method comprising administering a composition comprising an effective amount of the *Lactiplantibacillus plantarum* GBCC_F0070 strain deposited under Accession No. KCTC15051BP, a culture of the strain, a lysate of the strain, or a mixture thereof, to a subject in need thereof.

31. A use of a composition comprising the *Lactiplantibacillus plantarum* GBCC_F0070 strain deposited under Accession No. KCTC15051BP, a culture of the strain, a lysate of the strain, or a mixture thereof, for preparing a pharmaceutical preparation for preventing or treating an allergic disease.
